# EUROPEAN PATENT SPECIFICATION

(11) **EP 4 472 685 B1**
(45) Date of publication and mention of the grant of the patent: **02.12.2026**
(21) Application number: 23703526.6
(22) Date of filing: 31.01.2023
(51) Int. Cl.: A61L 27/36, A61L 27/46, A61L 27/50

(54) **MALLEABLE BONE REPAIR MATERIAL**
VERFORMBARES KNOCHENREPARATURMATERIAL
MATÉRIAU DE RÉPARATION OSSEUSE MALLÉABLE

(30) Priority: 31.01.2022 NL 2030793
(43) Date of publication of application: 11.12.2024
(73) Proprietor: Access2Bone IP B.V., 2333 BD Leiden (NL)
(72) Inventor: DE ROODE-BEULING, Nienke, 2333 BD Leiden (NL); SICKLER, Michael, 2333 BD Leiden (NL); VAN DE MORTEL, Arnoldus C.M., 2333 BD Leiden (NL)
(74) Representative: Nederlandsch Octrooibureau
(86) International application number: PCT/NL2023/050042
(87) International publication number: WO 2023/146406

(56) References cited:
- US-A1- 2006 051 427
- US-A1- 2008 033 572
- US-A1- 2008 188 946
- MINAYCHEV V V ET AL: "Aggregation of Nanosized Hydroxyapatite Particles and Inhibition of Cell Adhesion on this Bio-Active Material as Key Factors that Limit its Biointegration", BIOFIZIKA, PLEIADES PUBLISHING, MOSCOW, vol. 64, no. 5, 1 September 2019 (2019-09-01), pages 761 - 764, XP036966072, ISSN: 0006-3509, [retrieved on 20191216], DOI: 10.1134/S0006350919050154

## Description

### Field of the invention

The present invention relates to a bone repair material useful in the repair of bone defects. The invention further relates to malleable, non-settable, biocompatible bone repair materials that can be used for repair of bone defects.

### Background art

Every year, more than two million bone graft procedures are performed globally to address bone fractures and other orthopaedic-related injuries resulting from a variety of surgical, degenerative and traumatic causes. The repair of bone defects can be facilitated by placing a bone repair material in the defect site, where a loss of natural bone has occurred or where bone repair is impaired. The bone repair material is meant to selectively and over a sustained period of time promote the regeneration of natural bone structures.

The current clinical gold standard of treatment is autologous bone, harvested primarily from the patient's iliac crest or other locations such as the distal femur, proximal tibia, ribs, and intramedullary canal. Autologous bone provides an osteoconductive three-dimensional scaffold for bone growth, osteogenic cells and osteoinductive growth factors. Despite its immunocompatibility and excellent osteoconductive as well as osteoinductive properties, autograft bone is of limited supply and is often associated with limitations such as the need for additional surgery, pain at the donor site, morbidity, and a high and unpredictable resorption.

Alternatives are naturally derived bone repair materials, produced from cadaver bone. Naturally derived bone repair materials are usually prepared by acid extraction of most of the mineral contents resulting in so-called demineralized bone matrix (DBM). DBM has osteoinductive capabilities by the presence of natural growth factors and osteoconductive properties by the presence of a collagen matrix, the uncertainty of the availability limits its use though.

Another alternative bone repair material is a purely synthetic bone repair material. Synthetic bone repair materials based on hydroxyapatite and/or tricalcium phosphate (TCP) are widely used in oral surgery and orthopaedics. These synthetic bone repair materials are osteoconductive. The lack of osteoinductivity and osteogenic capabilities limit its use in patients with compromised bone regeneration. Examples are US 2008/188946 A1 which discloses a malleable bone repair material comprising DBM particles and a calcium phosphate powder, and US 2008/033572 A1 which discloses bone repair compositions comprising collagen, DBM and calcium phosphate particles such as betal-TCP.

To overcome the limitations of naturally derived bone repair materials and the purely synthetic bone repair materials a variety of other artificial or synthetic bone repair material is available on the market typically generally comprising a synthetic component and osteoinductive or osteogenic material such as growth factors.

A variety of growth factors have been shown to induce either cartilage or bone formation *in vivo.* For example, BMP-2 has been found to be feasible for use in the treatment of fractures and for use in bone regeneration. Certain recombinant osteogenic proteins have also been extensively studied and have demonstrated significant osteogenic activity in several models of bone formation.

It is now known that the production method of recombinant proteins is expensive and thus that bone repair products that comprise such recombinant factors are too costly.

It is further known that the combination products comprising a synthetic component and a growth factor has safety issues. Since recombinant growth factors used are generally single or a very limited type of growth factors in contrast to the whole spectrum of growth factors and proteins that naturally occur in bone overdosing of such specific factors often happens, a phenomenon associated with uncontrollable bone growth can occur.

Injectable and mouldable forms of bone substitute or repair material have been developed as they offer many advantages including increased handling ability during surgery and due to their ability to completely fill contained defects of complex geometric shapes. These bone substitutes currently mostly consist of a polymeric carrier, in which the polymeric carries prevents the cells to infiltrate into the bone defect.

Since bone tissues undergo constant tissue repair regulated by the mineral resorbing cells (osteoclasts) and mineral-producing cells (osteoblasts), the balance between osteoinductive factors such as those present in DBM and minerals and in particular calcium phosphate (CaP) is important in providing all building blocks for bone repair.

Thus, there is a continued need for alternative bone repair materials, that are effective, efficient and safe by design.

### Summary of the invention

The malleable bone repair material of the present invention meets the needs in the art by providing a material that is easy to handle and store, does not settle over time, displays both osteoconductive and osteoinductive properties, is free of polymeric carriers, is substantially bioabsorbable, and is safe by design. The bone repair material is provided as a malleable material consisting of a malleable demineralized bone matrix enriched with an osteoconductive material. The bone repair material may be delivered to a surgeon in a pre-loaded packaging, ready for use.

It has now been discovered by the inventors that a biological component rich in bioactive material and stabilized with an osteoconductive component provides for a malleable bone repair material for use in the treatment of bone defects.

The invention thus pertains to a malleable bone repair material suitable for bone defect repair, wherein the bone repair material composition consists of a biological component comprising bioactive material, preferably a malleable demineralized bone matrix that is enriched with an osteoconductive material. The malleable bone repair material provides for a biodegradable and stable composition with a biological component comprising bioactive material supplemented with the minerals essential for bone healing and growth, wherein the malleable bone repair material consists of: a) between 40 and 90 wt% of a biological component based on the total weight of the bone repair material; b) between 5 and 60 wt% of an osteoconductive component based on the total weight of the bone repair material; wherein the osteoconductive component comprises osteoconductive particles having a diameter between 10 nm and 300nm, wherein the osteoconductive particles do not react with one another in such a way that a setting reaction, wherein the bone repair material would set, takes place and c) optionally between 0.1 and 5 wt% of further osteoactive and/or stabilizing components, wherein the biological component comprises 0.3 to 6.0 wt% of bioactive material per gram of biological component and wherein the bioactive material consists of material derived from demineralized bone matrix [DBM] and is in the form of a gel or putty comprising DBM-derived collagen and proteins.

### Brief description of the drawings

The present invention will be discussed in more detail below, with reference to the attached drawing:
**Figure 1** shows malleable bone repair material with calcium phosphate particles.
**Figure 2** shows the malleable bone repair material according to the invention enhanced with calcium phosphate particles in a syringe.
**Figure 3** shows longitudinal and transverse µCT images of a partially remodeled bone graft after 4 weeks in a rabbit model.
**Figure 4** shows a µCT image of a completely remodeled bone graft 4 weeks after introduction of bone repair material in the femur of a rabbit.
**Figure 5** shows µCT images of bone defects in sheep 8 weeks after introduction of a malleable bone repair material with a high, medium or low amount of bioactive material, autologeous bone or no bone repair material.

### Description of embodiments

### Definitions

The term "malleable" as used in the context of the invention means that the material is capable of being converted from a first shape to a second shape by the application of pressure. The malleable bone repair material is configured to get any desired shape to fit a bone defect site. In some embodiments, the malleable material may be moulded (such as but not limited to being shaped by hand) to fit into a surgical site, such as a bone defect site. In some embodiments, the malleable material can be injected through a syringe directly into the target tissue site (e.g., bone defect, fracture, or void) into a wide variety of configurations. The malleable bone repair material according to the invention remains in the form of a paste and does not set i.e. does not become hard(er) over time and is, owing its malleable characteristics well bioresorbable.

As used herein, "long bone" refers to hard, dense bones that provide strength, structure, and mobility. A long bone has a shaft and two ends. Long bones typically are longer than they are wide. Examples of long bones include the femur, fibula, humerus, and tibia.

As used herein, "osteoconductive" or "mediates osteoconduction" refers to a scaffold property of a bone graft allowing for the ingrowth of neovasculature and the migration of osteoprogenitors into the graft site.

As used herein, "osteoinductive" or refers to the ability of the bone repair material to induce cell migration, proliferation, and/or differentiation of stem cells, osteoprogenitor cells, osteoblasts or osteocytes into mature bone cells. This process is mediated by bone growth factors or osteoinductive growth factors within the graft material.

As used herein, "bone growth factor" or "osteoinductive growth factor" refers to a growth factor or other proteins that supports osteoinduction. Examples of such growth factors include, but are not limited to, bone morphogenic proteins (e.g. BMP- 2, -4, -6, -7 or -9), basic fibroblast growth factor (bFGF), insulin-like growth factor-I and -II (IGF-I and IGF-II), platelet derived growth factor (PDGF) and transforming growth factor-betas (TGF-Ps).

As used herein, a "bone disease, disorder, defect or injury" refers to any bone condition in a subject resulting from cause or condition including, but not limited to, infections, acquired conditions, genetic conditions, or trauma. A disease, disorder, defect or injury of interest herein include any in need of bone growth and/or remodelling.

As used herein, the term "subject" refers to an animal, including a mammal, such as a human being.

The term "stabilizing compound" as used herein indicates a compound that provides improved cohesive characteristics, prevents drying out of the bone repair material over time, contains the moisture content and maintains the handability or manipulatability of the bone repair material over time. Stabilizing compounds according to the invention maintain the handling performance of the bone repair material performance and/or provide the bone repair material with a putty like consistency that is non runny and not sticky. Non limiting examples of such stabilizing compounds are hyaluronic acid, gelatin and glycerine.

### Bone repair material

In a first aspect, the invention pertains to a bone repair material comprising a biological component enriched with an osteoconductive component as defined by claims 1-14.

The biological component according to the invention comprises a solution of a bioactive material, which generally includes at least osteoinductive proteins such as, but not limited to, growth factors and/or a mixture of bioactive factors such as collagen extracted from bone, preferably from demineralized bone matrix. In the context of the invention said biological component of demineralized bone matrix is preferably malleable demineralized bone matrix.

The osteoconductive component in the bone repair material according to the invention is comprising essentially particles. The particles of the osteoconductive component have a diameter between 10nm to 300 nm.

The malleable bone repair material according to the invention is a shelf-stable bone repair material that remains malleable for at least 3 months, preferably at least 6 months, more preferably at least 12 months. In a preferred aspect the malleable bone repair material is shelf stable for at least 3 months, preferably at least 6 months, more preferably at least 12 months when stored at a temperature between about 1 to 10 °C, preferably between about 2 to 8 °C.

The malleable bone repair material was found to be malleable yet cohesive while not setting or curing over time. In a preferred embodiment the malleable bone repair material has a toothpaste to clay-like viscosity and keeps this viscosity over time. In a preferred embodiment the malleable bone repair material has and keeps a viscosity of about 70 mPa.s to about 400 Pa.s , and preferably, from 140 mPa.s to 300 Pa.s, and most preferably, from 600 mPa.s to 200 Pa.s, taken at 20 °C at a rotation speed of 20 revolutions per minute with a Brookfield Viscometer, Spindle No. 5. In the context of the invention, it is understood that the malleable bone repair material is shelf-stable and thus maintains a stable viscosity over time.

### Biological component

The biological component in the bone repair substrate of the invention comprises a bioactive material.

In one embodiment the bioactive materials for use in the bone repair substrate generally include human growth factors and collagen. In an embodiment the bioactive materials for use in the bone repair substrate are derived from allogeneic or xenogeneic bone, in a particularly preferred embodiment derived from demineralized bone matrix, preferably human demineralized bone matrix. In the context of the invention demineralized bone matrix in aqueous solution is considered malleable demineralized bone matrix.

Malleable demineralized bone matrix is demineralized bone matrix in a form that is capable of being converted from a first shape to a second shape by the application of pressure, such as, for example, in the form of a putty.

The presence of DBM is known to induce cellular recruitment to a site of injury where the recruited cells may eventually differentiate into bone forming cells Such recruitment of cells leads to an increase in the rate of healing and, therefore, to faster recovery for the patient.

It is believed that demineralized bone matrix provides for all factors to initiate, sustain as well as when needed inhibit bone growth.

The bioactive materials for use in the bone repair material and derived from allogeneic or xenogeneic bone are obtainable through methods as described herein and in the art in accordance with US 5,236,456. In an embodiment the bioactive material for use in the bone repair substrate may also comprise a mixture of bone derived bioactive materials and recombinant bioactive materials.

Demineralized bone matrix as used herein, refers to any material obtainable by removing mineral material from bone tissue according to conventional tissue bank techniques as described in Reddi et al. "Biochemical sequences in the transformation of normal fibroblasts in adolescent rats", Proc. Nat. Acad. Sci, 69 pp1601 - 5 (1972), Zhang et al. "Effect(s) of the Demineralization Process on the Osteoinductivity of Demineralized Bone Matrix" Journal of Periodontology, Volume 68, Issue 11 (1997) and according to US 6,189,537 and according to US 7,811,608. In a preferred embodiment, the demineralized bone matrix described herein include preparations containing less than 8% calcium and preferably not less than approximately 2% calcium by weight. Bioactive material, preferably demineralized bone matrix as used in the malleable bone repair material preferably has a calcium-content between 1 and 10 wt%, preferably between 2 and 8 wt% based on total weight of the demineralized bone matrix.

The bioactive material from bone may be provided in any suitable manner known in the art. Demineralized bone matrix products may serve as a source for extraction of bioactive material, which are also available commercially, including for instance, from AlloSource (Denver, CO, USA), KeraLink (Baltimore, Maryland), and others. The bone source may be selected from cortical, cancellous, or corticocancellous bone. Further the bone may be of allogeneic or xenogeneic origin, in a preferred embodiment the origin of the bone is human bone. Use of bioactive material from bone in tissue repair compositions is described in the art such as US 4,394,370, US 5,405,390, US 5,236,456 as well as WO2021005412 A1.

In a preferred embodiment the bioactive material is malleable demineralized bone matrix that may be extracted from demineralized bone matrix having a fiber and/or particle size smaller than about 1000 µm, more preferably smaller than about 900 µm, even more preferably smaller than about 850 µm. In a preferred embodiment the malleable bone repair material thus comprises malleable bioactive material derived from a demineralized bone matrix product as a starting material. The bioactive material consists of material derived from demineralized bone matrix and is in the form of a gel or putty comprising DBM-derived collagen and proteins. The bioactive materials in such a gel or putty are understood to no longer have a size range smaller than about 1000 µm yet are reduced to the molecular size ranges of the composition.

Bioactive material derived from allogeneic or xenogeneic bone, in particular bioactive material derived from demineralized bone matrix comprises a selection or all of the following factors: non-collagenous proteins including phosphoproteins, osteocalcin, matrix Gla protein; osteoinductive growth factors including bone morphogenic proteins (BMP) such as BMP-2, BMP-4, BMP-6 and BMP-7; factors that contribute to bone growth such as fibroblast growth factor-2 (FGF-2), insulin like growth factor-I and II (IGF-I and IGF-II), platelet derived growth factor (PDGF), and transforming growth factor beta-1 (TGF-beta 1) and further type I collagen and traces of calcium.

In a preferred embodiment the singular bioactive factors of the malleable demineralized bone matrix have a beneficially small size distribution, between 3 kDa and 800 kDa, more preferably between 3.2 and 700 kDa, even more preferably between 3.5 and 600 kDa.

In an embodiment some of the malleable demineralized bone matrix may be in an agglomerated state having a size of at most 850 µm.

Not only osteoinductive growth factors play an important role in bone remodelling. The regulation of osteoclasts that provide for bone growth inhibition is important for controlled bone formation. E3 ubiquitin ligases such as Smurf1, β-TrCP1, WWP1, and related adapter proteins (e.g., Schnurri 3) are known to regulate protein activity of RUNX2 or ATF4 by promoting their proteasomal-dependent degradation. (Garrett et al. J Clin Invest.2003, 111(11): 1771-1782.).Mixtures of such regulating factors are also present in the bioactive material derived from allogeneic or xenogeneic bone, in particular bioactive material derived from demineralized bone matrix.

The bioactive material comprises the whole spectrum of naturally derived proteins, which results in a controlled and physiological bone remodelling.

In a most preferred embodiment, the bioactive material comprises osteoinductive and osteoconductive factors derived from allogeneic or xenogeneic bone, in particular from demineralized bone matrix. In a particularly preferred embodiment, the biological material of the biological component is malleable demineralized bone matrix.

The biological component of the bone repair material typically comprises about 3000 to 60000 microgram per gram, preferably 4000 to 50000 microgram per gram, and even more preferably 6000 to 40000 microgram of bioactive material per gram biological component. In a preferred embodiment the biological component of the bone repair material comprises 3000 to 60000 microgram per gram, preferably 4000 to 50000 microgram per gram, and even more preferably 6000 to 40000 microgram of demineralized bone matrix per gram biological component. Alternatively worded, the biological component of the bone repair material comprises about 0.3 to 6 wt%, preferably 0.4 to 5 wt%, even more preferably 0.6 to 4 wt% of bioactive material per gram of biological component, preferably the bioactive material is demineralized bone matrix, most preferably malleable demineralized bone matrix.

The biological component of the bone repair component typically forms between 40 and 90 wt%, preferably between 45 and 87 wt%, more preferably between 50 and 85 wt% of the total weight of the bone repair material. In one aspect of the invention the bone repair material may comprise between 54 and 83 wt% of biological material by weight of the bone repair material.

### Osteoconductive Component

The osteoconductive component in the bone repair material is generally made of particles. The primary purpose of the osteoconductive component is to mediate osteoconduction. The osteoconductive particles of the osteoconductive component have a composition and architecture appropriate to remain in place and function as a carrier for the biological component. While these characteristics may vary between applications, exemplary compositions of the osteoconductive particles of the malleable bone repair substrate generally include particles comprising a synthetic osteoconductive matrix, such as a calcium phosphate-based matrix, a calcium sulphate-based matrix or bioglass matrix, or a human derived osteoconductive matrix, such as demineralized bone matrix powder, demineralized bone matrix fibers, cancellous bone matrix and bone chips. Essentially, in order for the bone repair material to be, and remain, malleable the osteoconductive particles used in the bone repair material according to the invention do not react with one another in such a way that a setting reaction, wherein the bone repair material would set, takes place.

When the particles of said osteoconductive component are in the form of a calcium phosphate-based matrix, said matrix may be selected from monocalcium phosphate monohydrate, dicalcium phosphate, dicalcium phosphate dehydrate, octocalcium phosphate, precipitated hydroxyapatite, precipitated amorphous calcium phosphate, monocalcium phosphate, alpha-tricalcium phosphate (α-TCP), beta-tricalcium phosphate (β-TCP), sintered hydroxyapatite, oxyapatite, tetracalcium phosphate, hydroxyapatite, calcium-deficient hydroxyapatite, and combinations thereof.

Worded alternatively calcium-phosphate-based particles of the osteoconductive component may comprise monocalcium phosphate monohydrate, dicalcium phosphate, dicalcium phosphate dehydrate, octocalcium phosphate, precipitated hydroxyapatite, precipitated amorphous calcium phosphate, monocalcium phosphate, alpha-tricalcium phosphate (α-TCP), beta-tricalcium phosphate (β-TCP), sintered hydroxyapatite, oxyapatite, tetracalcium phosphate, hydroxyapatite, calcium-deficient hydroxyapatite, or combinations thereof.

In the context of the invention when mixtures of calcium-phosphates are used such combinations preferably do not settle or cure. The use of calcium-phosphates that do not react with each other and set allows for the bone repair material to remain malleable and thereby allow for instant cell penetration in situ and good bone repair properties. Known mixtures or combinations that are not part of combinations that can be used in the osteoconductive component are combinations of calcium phosphates known in the art used to prepare cements such as tetra calcium phosphate in combination with any one of dicalcium phosphate dihydrate, dicalcium phosphate, anhydrous dicalcium phosphate and tricalcium phosphate or β-TCP in combination with any one of anhydrous dicalcium phosphate with calcium carbonate, dicalcium phosphate monohydrate, calcium sulphate or α-TCP in combination with any one of tetracalcium phosphate and dicalcium phosphate dihydrate. Setting properties of combinations of calcium phosphates are known in the art and can also be assessed by the skilled person by admixing combinations of calcium phosphates in the presence of an aqueous medium and assessing whether or not said combination sets.

The osteoconductive component of the malleable bone repair material preferably comprises osteoconductive particles, preferably having a diameter in the nanorange. It is believed that the use of nano-sized particles beneficially improves the cohesiveness and contribute to the stability of the malleable bone repair composition according to the invention. The osteoconductive particles have an average diameter between 10 and 300 nm, preferably between 25 and 250 nm, more preferably between 50 and 150 nm, even more preferably between 75 and 125 nm. In a preferred embodiment at least 65 wt%, preferably at least 70, more preferably at least 75 wt%, more preferably at least 80 wt%, even more preferably at least 90 wt% based on total weight of the osteoconductive particles are in the nanometer size-range. In an aspect of the invention essentially all osteoconductive particles are in the nanometer size-range. In an alternative embodiment the osteoconductive component of the malleable bone repair material may further comprise osteoconductive particles having a diameter in the micrometer size range. In an embodiment at most 35 wt% of the osteoconductive particles are in the micrometer range, preferably at most 30 wt%, more preferably at most 25 wt%, more preferably at most 20 wt%, most preferably at most 15 wt%. In an alternative embodiment the malleable bone repair material may further comprise between 0.01 wt% and 20 wt%, preferably between 0.1 wt% and 15 wt%, more preferably between 0.2 wt% and 10 wt%, more preferably between 0.5 wt% and 8 wt%, most preferably between 1 wt% and 5 wt% of osteoconductive particles having a diameter in the micrometer size range based on the total weight of the osteoconductive particles. The osteoconductive particles in the micrometer range preferably have an average diameter between 20 and 1,000 µm, preferably between 50 and 750 µm, more preferably between 100 and 500 µm. Diameters apply to the largest dimension of the particles. The diameter can be determined by methods known in the art using SEM or sieve analysis according to ISO13175-3.

Since nano-sized osteoconductive particles contribute to the cohesiveness and stability of the malleable bone repair material it is preferred that more than 50%, more preferably more than 75%, even more preferably more than 90%, most preferably essentially all of the total volume of the nano-sized particles is in singular or unagglomerated form. Agglomeration would impair the particles in providing their beneficial effect on the cohesiveness and/or stability of the malleable bone rapair material.

The osteoconductive particles are formed according to processes known in the art. The size of the particles is obtained using generally known processes.

The osteoconductive particles forming the osteoconductive component typically form between 5 and 60 wt%, preferably between 10 and 55 wt%, more preferably between 13 and 50 wt% of the total weight of the malleable bone repair material.

In one embodiment there is provided for the presence of particles in the bone repair material in both the nanosized ranged and the microsized range. It is believed that the addition of a mixture of nano- and micrometer sized particles to the bone repair material may provide for more structural integrity of the bone repair material.

### Bone repair material

In an embodiment of the invention the bone repair material is free of synthetic polymers and/or synthetic fillers, this provides the advantage that the bone repair material is not preventing the cells to penetrate the bone repair material, as is often the case when synthetic polymers are used.

In an embodiment the biological component of the bone repair component forms between 40 and 90 wt%, preferably between 45 and 87 wt%, more preferably between 50 and 85 wt% of the total weight of the bone repair material.

In one embodiment the bone repair material comprises a) between 40 and 90 wt%, preferably between 45 and 87 wt%, more preferably between 50 and 85 wt% of biological component based on the total weight of the bone repair material, b) between 5 and 60 wt%, preferably between 10 and 55 wt%, more preferably between 13 and 50 wt% osteoconductive component, preferably osteoconductive particles by weight of the total bone repair material and, c) optionally between 0.1 and 5wt%, preferably between 0.2 and 2 wt%, even more preferably between 0.5 and 1 wt% of further osteoactive and/or stabilizing components such as, but not limited to, hyaluronic acid, gelatin and glycerin.

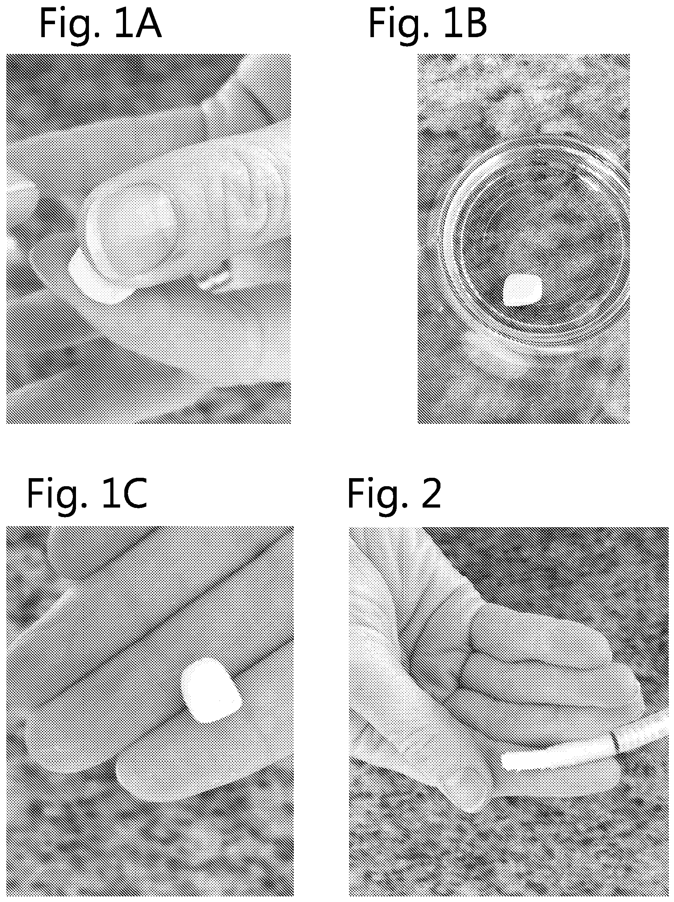

In one embodiment there is provided a syringe containing malleable bone repair material according to the invention. Figure 1 illustrates a malleable bone repair material. Figure 2 illustrates a syringe containing malleable bone repair material. In a preferred embodiment of the invention the syringe contains malleable bone repair material according to the invention comprising a) between 50 and 85 wt% of biological component based on the total weight of the bone repair material, the biological component preferably comprising 1 to 100 microgram of bioactive material per gram biological component, b) between 13 and 50 wt% of osteoconductive component based on the total weight of the bone repair material, the osteoconductive component preferably being osteoconductive particles, more preferably calcium phosphate particles having a diameter between 20 and 1000 nm.

The bone repair material optionally comprises osteoactive and/or stabilizing components such as, but not limited to hyaluronic acid, glycerine, carboxy methyl cellulose and gelatin. In the context of the invention the optional osteoactive and/or stabilizing component are not osteoconductive particles of the osteoconductive component. In one embodiment these components form between 0.1 and 5 wt%, preferably between 0.2 and 2 wt%, even more preferably between 0.5 and 1 wt% of the bone repair material. The weight percentage of the osteoactive and/or stabilizing components is not included in the weight of the biological component nor the osteoconductive component. It is believed that the addition of hyaluronic acid renders the bone repair material more malleable and prevents dehydration of the bone repair material thereby providing for a longer shelf-life. The addition of the further components in addition to the biological material of the biological component provides for a more viscous bone repair material. The use of (synthetic) polymers such as poloxamers is not advised since it is believed that such molecules inhibit cell penetration into the bone repair material.

It is believed that the bone repair material within these component ranges provides for a versatile bone repair material useful for a wide range of bone defects. In a particular embodiment the bone repair material is particularly suitable for the repair of long bone defects.

### Bone defects

In a preferred embodiment the malleable bone repair material of the invention is particularly useful in a wide variety of bone defects ranging from fractures in long bone, so-called non-union long-bone defects to bone defects caused by bone tumours or trauma. The malleable bone repair material is thus of particular use in non-load-bearing applications such as repair of contained bone defects.

### Treatment

The invention also provides the malleable bone repair material for use in therapeutic methods of treating bone defects in a subject in need thereof, as defined in claim 15. In an embodiment the method involves administering to a mammal having such bone fractures or defects a therapeutically effective amount of the bone repair material as described herein.

In one embodiment the method for treating a patient comprises the steps of contacting a bone tissue of the patient with the bone repair material.

In one embodiment the malleable bone repair material provides for an implantable device.

The bone repair material may be used to treat or prevent bone loss, promote union, or heal a fracture, and/or otherwise increase bone mass or treat a bone condition in the patient. Additionally, the bone repair material according to the invention facilitates the formation of new bone without impairing the natural healing process. The malleable bone repair material according to the invention beneficially is not a setting material, the malleable bone repair material therewith allows easy infiltration of cellular infiltrates involved in repair of the bone defect.

### Examples

### Example 1

Manufacturing example of bone repair material with round shaped osteoconductive particles
To prepare round-shaped particles the following steps were performed:
a. Dissolve Calcium Acetate Hydrate in Deionized water;
b. Heat the solution till >75 °C;
c. Slowly add a dilution of Phosphoric Acid;
d. Neutralize the solution by washing with Deionized water and water for injection (WFI).

### Example 2

Manufacturing example of bone repair material with rice shaped osteoconductive particles
a. Dissolve calcium nitrate in Deionized water;
b. Heat the solution till >75 °C;
c. Add ammonia 25% to the solution and keep pH of solution >8 during reaction;
d. Solution of Ammonium Phosphate in Deionized water is added slowly;
e. Age solution for at least 28 days;
f. Neutralize solution by washing with Deionized water and water for injection (WFI)

### Example 3

### Rabbit study

To evaluate the biological incorporation and remodeling into new bony structure of 2 bone repair materials with different Calcium Phosphate Nanoparticle formulas a rabbit study was undertaken Formula 1 was osteoconductive material based on HA (Calcium Nitrate + Ammonium Phosphate according to example 2), formula 2 on HA (Calcium Acetate + Phosphoric Acid according to example 1).

Nine rabbits underwent surgery to create a small round defect in each femoral condyle. The defect of the left femur was filled with material 1 and the defect of the right femur with material 2. After 28 days of follow-up (including weekly X-rays), rabbits were sacrificed, and femoral fragments were harvested and subjected to microCT.

**Table 1 Schematic Overview of the rabbit study**

| **Tested formula** | | 1: HA (Calcium Nitrate + Ammonium Phosphate) | | | | | |
|---|---|---|---|---|---|---|---|
| | | 2: HA (Calcium Acetate + Phosphoric Acid ) | | | | | |
| **Test system** | | Rabbit | | | | | |
| **Number of animals** | | 8 +1 reserve animal | | | | | |
| **Groups** | | 2 | | | | | |
| | | Left leg of the 8 rabbits: material 1 | | | | | |
| | | Right leg of the 8 rabbits: material 2 | | | | | |
| **Follow-up time** | | | 28 days | | | | |

| **Outcome parameters** | | Biological incorporation, remodeling into new bony structure and resorption of Calcium Phosphate Nanoparticles | | | | | |
|---|---|---|---|---|---|---|---|
| Time points | | | | | | | |
| | Description | Before surgery | | **D0** | **D1-5** | **D5--D28** | **D28** |
| | Clinical observations (incl T and BW) | x | | After surgery | Daily | Weekly | Before sacrifice (1) or post mortem(2) |
| | X-rays | x | | | x* | x | x¹ |
| | Necropsy | | | x | | x | x² |
| | Micro CT | | | | | | x² |

| | | | | | | | |
|---|---|---|---|---|---|---|---|
| *First 5 days post-operation: daily health inspection and administration of antibiotics & analgesics (typically following successful surgery, animals will not show lameness) | | | | | | | |

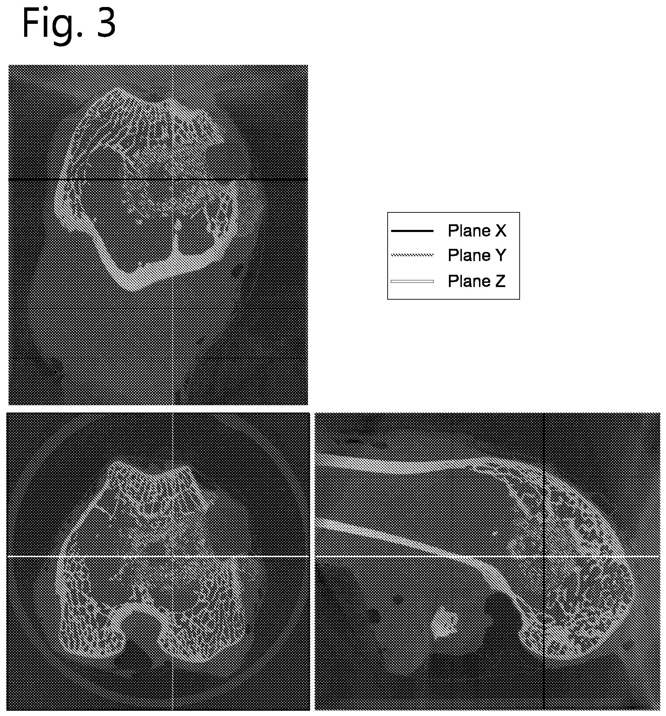

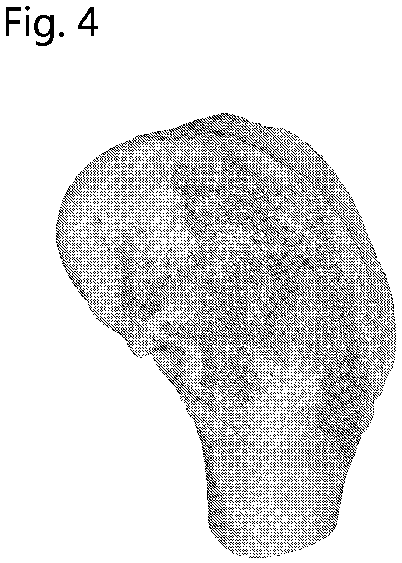

The calcium acetate based formula according to example 1 was judged as performing slightly better than the other formula based on qualitative observation of the µCT images at experimental day 28. Figure 3 illustrates longitudinal and transverse µCT images of a partially remodeled bone graft wherein the lines illustrate the section planes in the different images respectively. Figure 4 illustrates a 3D render of a completely remodeled bone graft treated with the bone repair material according to example 1. The bone defect is visibly partially filled with new bone, and the bone repair material has been partially resorbed.

### Example 4

### Malleable bone repair material with high concentration of biological component.

To prepare a malleable bone repair material with a high concentration of biological component 100 mg of Demineralized Bone was suspended in 1093 mg water, to achieve 1193 mg of the biological component. 402 mg of nano-sized low crystalline hydroxyapatite particles, to form a malleable bone repair material.

### Malleable bone repair material with medium concentration of biological component.

To prepare a malleable bone repair material with a medium concentration of biological component 25 mg of Demineralized Bone was suspended in 1063 mg water, to achieve 1088 mg of the biological component. 402 mg of nano-sized low crystalline hydroxyapatite particles, to form a malleable DBM.

### Malleable bone repair material with a low concentration of biological component.

To prepare a malleable bone repair material with a low concentration of biological component 3 mg of Demineralized Bone was suspended in 1063 mg water, to achieve 1066 mg of the biological component. 402 mg of nano-sized low crystalline hydroxyapatite particles, to form a malleable DBM.

Formulations of Malleable bone repair material (comprising sheep bone derived protein-based bioactive material, enhanced with osteoconductive Calcium Phosphate nanoparticles according to the invention with three different concentrations of the biological component) was evaluated on biological incorporation, remodelling, and resorption in a critical size defect model in sheep.

The Malleable bone repair material was also assessed for its osteoinductive capacity using C₂C₁₂ analysis on BMP2.

The aim of this study was to assess biological incorporation, remodelling into new bony structure and resorption of the Malleable DBM (as compared to standard of care).

The experiment was performed with 10 sheep in total, each sheep was exposed to critical size drilled bone defects (6 defects per sheep)
4 groups of sheep:
   Group 1: positive control group (autologous bone from sheep)
   Group 2: Malleable DBM at high concentration
   Group 3: Malleable DBM at medium concentration
   Group 4: Malleable DBM at low concentration
   Group 5: negative control group (empty defect)
2 time points for follow-up:
   4 weeks
   8 weeks
6 defects per group/timepoint

### Results

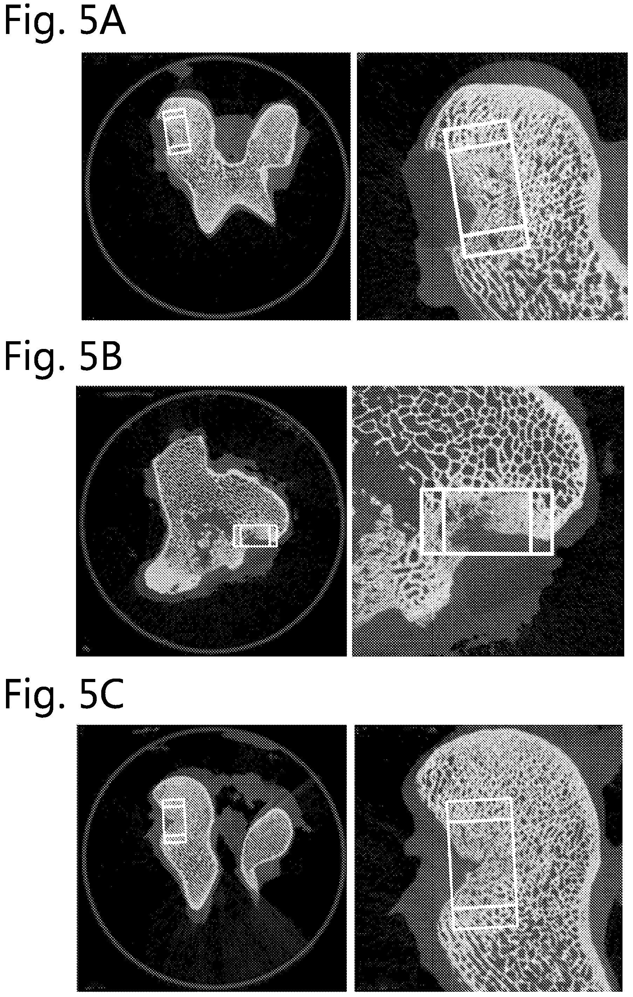

Exemplary defect TD351_31 was filled with about 1 ml of the bone repair material with a high concentration of biological active ingredient. After 8 weeks the area where the defect was located, was analyzed using micro-CT. In figure 5A it can be seen that the defect was almost completely filled with bone. Compared to the surrounding bone the bone volume was 94%.

Exemplary defect TD351_13 was filled with about 1 ml of the bone repair material with a medium concentration of biological active ingredient. After 8 weeks the area where the defect was located, was analyzed with micro-CT. In figure 5B it can be seen that most part of the defect was filled with bone. Compared to the surrounding bone the bone volume was about 80%.

Exemplary defect TD351_54 was filled with about 1 ml of the bone repair material with a low concentration of biological active ingredient). After 8 weeks the area where the defect was located, was analyzed with micro-CT. In figure 5C it can be seen that most part of the defect was filled with bone. Compared to the surrounding bone the bone volume was about 80%.

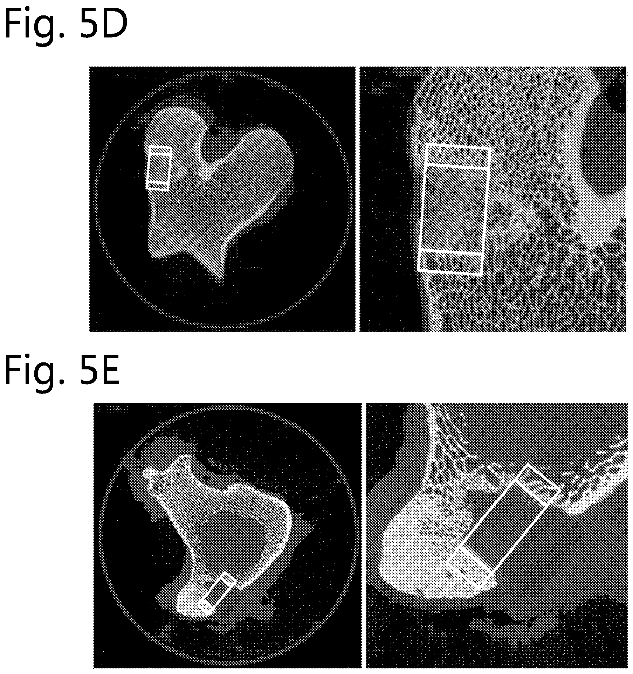

As a control two further groups of sheep were taken into account. As the positive control a bone defect was filled with autologous bone from the sheep. Exemplary defect TD351_15 was filled with autologous bone. After 8 weeks the area where the defect was located, was analysed with micro-CT. In figure 5D it can be seen that the defect was almost completely filled with bone. Compared to the surrounding bone the bone volume was 98%.

As a negative control a bone defect was left unfilled with any material. Exemplary defect TD351_07 was not filled. After 8 weeks the area where the defect was located, was analysed with micro-CT. In figure 5E it is visible that the bone defect was still empty. Compared to the surrounding bone the bone volume was only 18%.

In addition to the *in vivo* behaviour of the bone repair material ALP induced by BMP-2 was assessed in C2C12 cells by determination of the ALP index (OI) with bone repair material with a high and a medium concentration of bioactive material.

The osteoinductivity of the bone morphogenetic proteins (BMPs), present in the bone repair material, was measured in vitro using a pluripotent myoblast C2C12 cell line by the method described in Han B et al. J. Orthopedic Research, vol 21(4), 2003; pages 648 - 654. Alkaline phosphatase activity induced by co-incubation of DBM with C2C12 cells was dose-responsive and corresponds to the quantity of active BMPs in DBM.

**Table 2. ALP index of 2 bone repair materials.**

| **Sample** | **ALP induced by DBM in C2C12 Cells** |
|---|---|
| | **ALP index (OI)** |
| Bone repair material with a high concentration of biological active ingredient | 0.411 |
| Bone repair material with a medium concentration of biological active ingredient | 0.405 |

Based on the results obtained with the in vitro and in vivo correlation studies, a bone repair material with an ALP index of more than 20% (0.20) above a (negative) baseline was considered osteoinductivity positive.

### Example 5

To assess the shelf-life of the malleable bone repair material a batch of malleable bone repair material with high concentration of biological component according to example 4 was prepared. The bone repair material was put into syringes and stored at a temperature of 4 - 8 °C. The malleability of the bone repair material was assessed after storage for 6, 17 and 45 weeks. At all 3 time points the bone repair material could be pushed out of the syringe and moulded manually into a sphere.

## Claims

1. A malleable bone repair material consisting of:
a) between 40 and 90 wt% of a biological component based on the total weight of the bone repair material;
b) between 5 and 60 wt% of an osteoconductive component based on the total weight of the bone repair material; wherein the osteoconductive component comprises osteoconductive particles having a diameter between 10 nm and 300nm, wherein the osteoconductive particles do not react with one another in such a way that a setting reaction, wherein the bone repair material would set, takes place
c) and optionally between 0.1 and 5 wt% of further osteoactive and/or stabilizing components,
wherein the biological component comprises 0.3 to 6.0 wt% of bioactive material per gram of biological component and wherein the bioactive material consists of material derived from demineralized bone matrix [DBM] and is in the form of a gel or putty comprising DBM-derived collagen and proteins.

2. Malleable bone repair material according to claim 1, wherein the bone repair material is shelf-stable for at least 3 months at a temperature between 1 and 10 °C.

3. Malleable bone repair material according to the preceding claims wherein the osteoconductive component comprises osteoconductive particles having a diameter between 10 nm and 300nm, wherein more than 50% of the total volume of the nano-sized particles is in singular or unagglomerated form.

4. Malleable bone repair material according to the preceding claims wherein the biological component comprises 1 to 100 microgram of bioactive material per gram of biological component.

5. Malleable bone repair material according to the preceding claims wherein the bone repair material is non-settable.

6. Malleable bone repair material according to the preceding claims wherein the osteoconductive component comprises osteoconductive particles having a diameter between 20 µm and 1000µm.

7. Malleable bone repair material according to the preceding claims wherein the osteoconductive component comprises osteoconductive particles being a mixture of nano sized particles having a diameter between 10 nm and 300nm and micron sized particles having a diameter between 20 µm and 1000µm.

8. Malleable bone repair material according to the preceding claims, wherein the bone repair material comprises further osteoactive and/or stabilizing components selected from hyaluronic acid, glycerine, carboxy methyl cellulose, gelatin and mixtures thereof.

9. Malleable bone repair material according to the preceding claims wherein the osteoconductive component consists of osteoconductive particles, preferably calcium phosphate-based particles.

10. Malleable bone repair material according to the preceding claims wherein the calcium phosphate-based particles comprise monocalcium phosphate monohydrate, dicalcium phosphate, dicalcium phosphate dehydrate, octocalcium phosphate, precipitated hydroxyapatite, precipitated amorphous calcium phosphate, monocalcium phosphate, alpha-tricalcium phosphate (α-TCP), beta-tricalcium phosphate (β-TCP), sintered hydroxyapatite, oxyapatite, tetracalcium phosphate, hydroxyapatite, calcium-deficient hydroxyapatite, or mixtures thereof.

11. Malleable bone repair material according to any one of the previous claims wherein the osteoconductive component comprises calcium-sulphate particles and/or bioglass particles.

12. Malleable bone repair material according to any one of the previous claims wherein the osteoconductive component comprises particles obtained from human donor bone, such as demineralized bone matrix powder, cancellous bone, bone chips, demineralized bone matrix fibres and the like.

13. Malleable bone repair material according to any one of the previous claims wherein the bone repair material is configured to fit at or near a bone defect to promote bone union and/or growth.

14. Malleable bone repair material according to any one of the previous claims wherein the bone repair material is in the form of a malleable putty or paste and packaged in a capped syringe.

15. Malleable bone repair material for use in the treatment of bone defects in a subject in need thereof wherein the bone repair material comprises
a) between 40 and 90 wt% of a biological component based on the total weight of the bone repair material;
b) between 5 and 60 wt% of an osteoconductive component based on the total weight of the bone repair material; wherein the osteoconductive component comprises osteoconductive particles having a diameter between 10 nm and 300nm, wherein the osteoconductive particles do not react with one another in such a way that a setting reaction, wherein the bone repair material would set, takes place
c) and optionally between 0.1 and 5 wt% of further osteoactive and/or stabilizing components,
wherein the biological component comprises 0.3 to 6.0 wt% of bioactive material per gram of biological component and wherein the bioactive material consists of material derived from demineralized bone matrix [DBM] and is in the form of a gel or putty comprising DBM-derived collagen and proteins.

## Patentansprüche

1. Formbares Knochenreparaturmaterial, bestehend aus:
a) zwischen 40 und 90 Gew.-% einer biologischen Komponente, bezogen auf das Gesamtgewicht des Knochenreparaturmaterials;
b) zwischen 5 und 60 Gew.-% einer osteokonduktiven Komponente, bezogen auf das Gesamtgewicht des Knochenreparaturmaterials; wobei die osteokonduktive Komponente osteokonduktive Partikel mit einem Durchmesser zwischen 10 nm und 300 nm umfasst, wobei die osteokonduktiven Partikel nicht in einer solchen Weise miteinander reagieren, dass eine Abbindereaktion, wobei das Knochenreparaturmaterial abbinden würde, stattfindet
c) und optional zwischen 0,1 und 5 Gew.-% weiterer osteoaktiver und/oder stabilisierender Komponenten,
wobei die biologische Komponente 0,3 bis 6,0 Gew.-% bioaktives Material pro Gramm biologischer Komponente umfasst und wobei das bioaktive Material aus von demineralisierter Knochenmatrix [DBM] abgeleitetem Material besteht und in Form eines Gels oder einer Knetmasse vorliegt, umfassend von DBM abgeleitetes Kollagen und Proteine.

2. Formbares Knochenreparaturmaterial nach Anspruch 1, wobei das Knochenreparaturmaterial für mindestens 3 Monate bei einer Temperatur zwischen 1 und 10 °C lagerstabil ist.

3. Formbares Knochenreparaturmaterial nach den vorhergehenden Ansprüchen, wobei die osteokonduktive Komponente osteokonduktive Partikel mit einem Durchmesser zwischen 10 nm und 300 nm umfasst, wobei mehr als 50 % des Gesamtvolumens der nanogroßen Partikel in vereinzelter oder nicht agglomerierter Form vorliegt.

4. Formbares Knochenreparaturmaterial nach den vorhergehenden Ansprüchen, wobei die biologische Komponente 1 bis 100 Mikrogramm bioaktives Material pro Gramm biologischer Komponente umfasst.

5. Formbares Knochenreparaturmaterial nach den vorhergehenden Ansprüchen, wobei das Knochenreparaturmaterial nicht abbindend ist.

6. Formbares Knochenreparaturmaterial nach den vorhergehenden Ansprüchen, wobei die osteokonduktive Komponente osteokonduktive Partikel mit einem Durchmesser zwischen 20 µm und 1000 µm umfasst.

7. Formbares Knochenreparaturmaterial nach den vorhergehenden Ansprüchen, wobei die osteokonduktive Komponente osteokonduktive Partikel umfasst, die ein Gemisch aus nanogroßen Partikeln mit einem Durchmesser zwischen 10 nm und 300 nm und mikrometergroßen Partikeln mit einem Durchmesser zwischen 20 µm und 1000 µm sind.

8. Formbares Knochenreparaturmaterial nach den vorhergehenden Ansprüchen, wobei das Knochenreparaturmaterial weitere osteoaktive und/oder stabilisierende Komponenten umfasst, ausgewählt aus Hyaluronsäure, Glycerin, Carboxymethylcellulose, Gelatine und Gemischen davon.

9. Formbares Knochenreparaturmaterial nach den vorhergehenden Ansprüchen, wobei die osteokonduktive Komponente aus osteokonduktiven Partikeln besteht, vorzugsweise Calciumphosphat-basierten Partikeln.

10. Formbares Knochenreparaturmaterial nach den vorhergehenden Ansprüchen, wobei die Calciumphosphat-basierten Partikel Monocalciumphosphatmonohydrat, Dicalciumphosphat, Dicalciumphosphatdihydrat, Octacalciumphosphat, gefälltes Hydroxyapatit, gefälltes amorphes Calciumphosphat, Monocalciumphosphat, alpha-Tricalciumphosphat (α-TCP), beta-Tricalciumphosphat (β-TCP), gesintertes Hydroxyapatit, Oxyapatit, Tetracalciumphosphat, Hydroxyapatit, calciumdefizitäres Hydroxyapatit oder Gemische davon umfassen.

11. Formbares Knochenreparaturmaterial nach einem der vorhergehenden Ansprüche, wobei die osteokonduktive Komponente Calciumsulfatpartikel und/oder Bioglaspartikel umfasst.

12. Formbares Knochenreparaturmaterial nach einem der vorhergehenden Ansprüche, wobei die osteokonduktive Komponente Partikel umfasst, die von menschlichem Spenderknochen gewonnen werden, wie Pulver aus demineralisierter Knochenmatrix, Spongiosa, Knochenspäne, Fasern aus demineralisierter Knochenmatrix und dergleichen.

13. Formbares Knochenreparaturmaterial nach einem der vorhergehenden Ansprüche, wobei das Knochenreparaturmaterial dazu konfiguriert ist, an oder in der Nähe eines Knochendefekts angepasst zu werden, um Knochenvereinigung und/oder Knochenwachstum zu fördern.

14. Formbares Knochenreparaturmaterial nach einem der vorhergehenden Ansprüche, wobei das Knochenreparaturmaterial in Form einer formbaren Knetmasse oder Paste vorliegt und in einer Spritze mit Verschlusskappe verpackt ist.

15. Formbares Knochenreparaturmaterial zur Verwendung bei der Behandlung von Knochendefekten bei einem Subjekt, das dessen bedarf, wobei das Knochenreparaturmaterial
a) zwischen 40 und 90 Gew.-% einer biologischen Komponente, bezogen auf das Gesamtgewicht des Knochenreparaturmaterials;
b) zwischen 5 und 60 Gew.-% einer osteokonduktiven Komponente, bezogen auf das Gesamtgewicht des Knochenreparaturmaterials; wobei die osteokonduktive Komponente osteokonduktive Partikel mit einem Durchmesser zwischen 10 nm und 300 nm umfasst, wobei die osteokonduktiven Partikel nicht in einer solchen Weise miteinander reagieren, dass eine Abbindereaktion, wobei das Knochenreparaturmaterial abbinden würde, stattfindet
c) und optional zwischen 0,1 und 5 Gew.-% weiterer osteoaktiver und/oder stabilisierender Komponenten,
umfasst, wobei die biologische Komponente 0,3 bis 6,0 Gew.-% bioaktives Material pro Gramm biologischer Komponente umfasst und wobei das bioaktive Material aus von demineralisierter Knochenmatrix [DBM] abgeleitetem Material besteht und in Form eines Gels oder einer Knetmasse vorliegt, umfassend von DBM abgeleitetes Kollagen und Proteine.

## Revendications

1. Matériau de réparation osseuse malléable consistant en :
a) entre 40 et 90 % en poids d'un composant biologique sur la base du poids total du matériau de réparation osseuse ;
b) entre 5 et 60 % en poids d'un composant ostéoconducteur sur la base du poids total du matériau de réparation osseuse ; dans lequel le composant ostéoconducteur comprend des particules ostéoconductrices ayant un diamètre entre 10 nm et 300 nm, dans lesquelles les particules ostéoconductrices ne réagissent pas entre elles de telle manière qu'une réaction de prise, dans laquelle le matériau de réparation osseuse ferait prise, ait lieu
c) et optionnellement entre 0,1 et 5 % en poids de composants ostéoactifs et/ou stabilisants supplémentaires,
dans lequel le composant biologique comprend 0,3 à 6,0 % en poids de matériau bioactif par gramme de composant biologique et dans lequel le matériau bioactif consiste en un matériau dérivé de la matrice osseuse déminéralisée [DBM] et se présente sous forme d'un gel ou d'un mastic comprenant du collagène et des protéines dérivés du DBM.

2. Matériau de réparation osseuse malléable selon la revendication 1, dans lequel le matériau de réparation osseuse est stable à la conservation pendant au moins 3 mois à une température entre 1 et 10 °C.

3. Matériau de réparation osseuse malléable selon les revendications précédentes dans lequel le composant ostéoconducteur comprend des particules ostéoconductrices ayant un diamètre entre 10 nm et 300 nm, dans lequel plus de 50 % du volume total des particules de taille nanométrique se trouve sous forme individuelle ou non agglomérée.

4. Matériau de réparation osseuse malléable selon les revendications précédentes dans lequel le composant biologique comprend 1 à 100 microgrammes de matériau bioactif par gramme de composant biologique.

5. Matériau de réparation osseuse malléable selon les revendications précédentes dans lequel le matériau de réparation osseuse est non durcissable.

6. Matériau de réparation osseuse malléable selon les revendications précédentes dans lequel le composant ostéoconducteur comprend des particules ostéoconductrices ayant un diamètre entre 20 µm et 1000 µm.

7. Matériau de réparation osseuse malléable selon les revendications précédentes dans lequel le composant ostéoconducteur comprend des particules ostéoconductrices étant un mélange de particules de taille nanométrique ayant un diamètre entre 10 nm et 300 nm et de particules de taille micronique ayant un diamètre entre 20 µm et 1000 µm.

8. Matériau de réparation osseuse malléable selon les revendications précédentes, dans lequel le matériau de réparation osseuse comprend des composants ostéoactifs et/ou stabilisants supplémentaires choisis parmi l'acide hyaluronique, la glycérine, la carboxyméthylcellulose, la gélatine et leurs mélanges.

9. Matériau de réparation osseuse malléable selon les revendications précédentes dans lequel le composant ostéoconducteur consiste en des particules ostéoconductrices, de préférence des particules à base de phosphate de calcium.

10. Matériau de réparation osseuse malléable selon les revendications précédentes dans lequel les particules à base de phosphate de calcium comprennent du phosphate monocalcique monohydraté, du phosphate dicalcique, du phosphate dicalcique dihydraté, du phosphate octocalcique, de l'hydroxyapatite précipitée, du phosphate de calcium amorphe précipité, du phosphate monocalcique, du phosphate tricalcique alpha (α-TCP), du phosphate tricalcique bêta (β-TCP), de l'hydroxyapatite frittée, de l'oxyapatite, du phosphate tétracalcique, de l'hydroxyapatite, de l'hydroxyapatite déficiente en calcium, ou leurs mélanges.

11. Matériau de réparation osseuse malléable selon l'une quelconque des revendications précédentes dans lequel le composant ostéoconducteur comprend des particules de sulfate de calcium et/ou des particules de bioverre.

12. Matériau de réparation osseuse malléable selon l'une quelconque des revendications précédentes dans lequel le composant ostéoconducteur comprend des particules obtenues à partir d'os de donneur humain, telles que de la poudre de matrice osseuse déminéralisée, de l'os spongieux, des copeaux osseux, des fibres de matrice osseuse déminéralisée et analogues.

13. Matériau de réparation osseuse malléable selon l'une quelconque des revendications précédentes dans lequel le matériau de réparation osseuse est configuré pour s'adapter au niveau ou à proximité d'un défaut osseux afin de favoriser l'union osseuse et/ou la croissance osseuse.

14. Matériau de réparation osseuse malléable selon l'une quelconque des revendications précédentes dans lequel le matériau de réparation osseuse se présente sous forme d'un mastic ou d'une pâte malléable et est conditionné dans une seringue munie d'un capuchon.

15. Matériau de réparation osseuse malléable destiné à être utilisé dans le traitement de défauts osseux chez un sujet qui en a besoin dans lequel le matériau de réparation osseuse comprend
a) entre 40 et 90 % en poids d'un composant biologique sur la base du poids total du matériau de réparation osseuse ;
b) entre 5 et 60 % en poids d'un composant ostéoconducteur sur la base du poids total du matériau de réparation osseuse ; dans lequel le composant ostéoconducteur comprend des particules ostéoconductrices ayant un diamètre entre 10 nm et 300 nm, dans lesquelles les particules ostéoconductrices ne réagissent pas entre elles de telle manière qu'une réaction de prise, dans laquelle le matériau de réparation osseuse ferait prise, ait lieu
c) et optionnellement entre 0,1 et 5 % en poids de composants ostéoactifs et/ou stabilisants supplémentaires,
dans lequel le composant biologique comprend 0,3 à 6,0 % en poids de matériau bioactif par gramme de composant biologique et dans lequel le matériau bioactif consiste en un matériau dérivé de la matrice osseuse déminéralisée [DBM] et se présente sous forme d'un gel ou d'un mastic comprenant du collagène et des protéines dérivés du DBM.
